(19) Europäisches Patentamt
European Patent Office
Office européen des brevets

(11) Publication number: **0 319 123 B1**

(12) **EUROPEAN PATENT SPECIFICATION**

(45) Date of publication of patent specification: **17.05.95**

(51) Int. Cl.[6]: **C12P 7/64**, C12P 5/02, C12P 7/04, C12P 13/00

(21) Application number: **88308091.3**

(22) Date of filing: **01.09.88**

# (54) Process for producing unsaturated fatty acid or unsaturated hydrocarbon.

(30) Priority: **30.11.87 JP 300057/87**
**30.11.87 JP 300058/87**

(43) Date of publication of application:
**07.06.89 Bulletin 89/23**

(45) Publication of the grant of the patent:
**17.05.95 Bulletin 95/20**

(84) Designated Contracting States:
**BE CH DE FR GB LI NL SE**

(56) References cited:

**JOURNAL OF BIOLOGICAL CHEMISTRY, vol. 251, no. 23, 10 December 1976, US; M.J. KOROLY et al., pp. 7588-7592&NUM;**

**CHEMICAL ABSTRACTS, vol. 105, no. 1, 07 July 1986, Columbus, OH (US); N.S. EGOROV et al., p. 309, no. 3180f&NUM;**

**CHEMICAL ABSTRACTS, vol. 98, no. 5, 31 January 1983, Columbus, OH (US); p. 537, no. 33069f&NUM;**

(73) Proprietor: **The Agency of Industrial Science and Technology**
**3-1, Kasumigaseki 1-chome**
**Chiyoda-ku**
**Tokyo-to (JP)**

(72) Inventor: **Itoh, Susumu**
**3441-64, Higashiminecho**
**Utsunomiya-shi**
**Tochigi (JP)**
Inventor: **Koike, Kenzo**
**11-14, Nakatomatsuri 1-chome**
**Utsunomiya-shi**
**Tochigi (JP)**
Inventor: **Takaiwa, Mikio**
**2606-6, Akabane**
**Ichikai-machi**
**Haga-gun**
**Tochigi (JP)**

(74) Representative: **Bannerman, David Gardner et al**
**Withers & Rogers**
**4 Dyer's Buildings**
**Holborn**
**London, EC1N 2JT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

**PATENT ABSTRACTS OF JAPAN**, vol. 12, no. 491 (C-554)[3338], 21 December 1988&NUM;

**PATENT ABSTRACTS OF JAPAN**, vol. 12, no. 491 (C-554)[3338], 21 December 1988&NUM;

**CHEMICAL ABSTRACTS**, vol. 111, no. 11, 11 September 1989, Columbus, OH (US); p. 602, no. 95627g&NUM;

**PATENT ABSTRACTS OF JAPAN**, vol. 12, no. 491 (C-554)[3338], 21 December 1988&NUM;

## Description

The invention relates to a process for producing an unsaturated fatty acid or an unsaturated hydrocarbon or a derivative thereof, utilising a microorganism.

Unsaturated fatty acids and derivatives thereof are widely used in, for example, perfumes, drugs, coatings, surfactants and cosmetics as well as starting materials for producing these products.

Known processes for preparing such unsaturated fatty acids and derivatives thereof involve hydrolysis of animal or vegetable fats, or chemical synthesis.

However the hydrolysis of a fat results in a mixture of unsaturated fatty acids having various chain lengths, whilst chemical synthesis requires a number of steps and results in a mixture of cis and trans stereoisomers. Both of these processes are disadvantageous in that the mixtures can be separated only with difficulty.

On the other hand, some methods for the production of unsaturated acids are known which involve the use of microorganisms. For example, Japanese Patent Publications Nos.37096/1986 and 22199/1983 have disclosed fermentation methods for the production of γ-linolenic acid involving microorganisms of the genera Thamnidium and Mortierella, respectively. However, each of these processes is disadvantageous in that the yield is low, that the reaction is slow and that the desired unsaturated fatty acid is accumulated within the microorganism cells and thus is difficult to recover.

Acyl-CoA desaturase (E.C. 1.14.99.5) is known to be one of enzymes which desaturate fatty acid derivatives. It originates from microorganisms, animals and plants. However the low activity and extremely low stability of this enzyme make it unsuitable for industrial application.

Unsaturated hydrocarbons and derivatives thereof are widely used in for example, pheromones, perfumes and drugs as well as starting materials for producing these products and intermediates in the synthesis of organic compounds.

Known processes for preparing such unsaturated hydrocarbons and derivatives thereof involve chemical synthesis by metathesis.

However such processes are disadvantageous in that they require a number of steps and result in a mixture of cis and trans stereoisomers.

U.S. Patent No. 3,629,072 discloses a process for the production of an alkene with the use of a microorganism belonging to the genus Nocardia. However this process is disadvantageous in that it requires a prolonged reaction period and gives a low yield.

Accordingly although it has been urgently required to establish a process for readily producing a dialkene from a monoalkene, which is easily available on an industrial scale, no process satisfying such a requirement has been proposed so far.

We have now found that a particular microorganism, which has been isolated from soil and subjected to mutation as will be shown in Referential Example 1 hereinafter, can convert a fatty acid or a hydrocarbon into the corresponding unsaturated fatty acid or hydrocarbon and that the unsaturated fatty acid or hydrocarbon thus produced is excreted from the cells and thus can be readily recovered. We have further found that the ability of said strain to producing an unsaturated fatty acid or hydrocarbon can be greatly enhanced by adding an amino acid such as L-glutamic or L-aspartic acid or a carbohydrate such as glucose to the reaction mixture comprising the strain and the substrate, i.e. a fatty acid or hydrocarbon, and further adding thiamine or an inorganic metal salt such as magnesium sulfate or manganese sulfate thereto.

The invention provides a process for preparing an unsaturated fatty acid, a derivative thereof, an unsaturated hydrocarbon or a derivative thereof from a fatty acid, a derivative thereof, a hydrocarbon or a derivative thereof, respectively, utilising a bacterium belonging to the genus Rhodococcus which is capable of producing an unsaturated fatty acid or an unsaturated hydrocarbon in the presence of a carbohydrate or an amino acid, characterized by conducting the reaction in the pressure of thiamine or an inorganic metal salt, as specified in claims 1 - 7.

According to a first aspect, the present invention provides a process for the production of an unsaturated fatty acid or a derivative thereof which comprises supplying a fatty acid or a derivative thereof to an unsaturated fatty acid-producing bacterium belonging to the genus Rhodococcus to give desired unsaturated fatty acid or a derivative thereof, characterised in that thiamine or an inorganic metal salt is added to the reaction system.

According to a second aspect, the present invention provides a process for the production of an unsaturated hydrocarbon or a derivative thereof which comprises supplying a hydrocarbon or a derivative thereof to an unsaturated hydrocarbon-producing bacterium belonging to the genus Rhodococcus to give the desired unsaturated hydrocarbon or a derivative thereof, characterized in that thiamine or an inorganic metal salt is added to the reaction system.

The bacterium to be used in the invention is obtained by subjecting a strain isolated from soil of Okinawa, Japan to a mutation treatment as shown in Referential Example 1. The mycological properties of this strain are as follows.

(A) Morphological properties

It is a bacillus having polymorphic cells. Namely, the cells are bacillary in a young culture and coccobacillary in an aged culture. A cell is 0.5 to 0.8 µm x 1.0 to 5.0 µm in size.

(B) Growth on various media

(1) Sucrose-nitrate agar medium

It grows poorly. Colonies are of a pale flesh color, smooth and slightly glossy.

(2) Glucose-asparagine agar medium

It grows poorly. Colonies are of a pale flesh color, smooth and slightly glossy.

(3) Glycerol-asparagine agar medium

It grows moderately well. Colonies are of a milky white color and slightly glossy. Some colonies are smooth while others are rough.

(4) Starch agar medium

It grows moderately well. Colonies are of a milky white color, smooth and slightly glossy.

(5) Tyrosine agar medium

It grows vigorously. Colonies are of a flesh color, smooth and glossy. Some colonies are slimy.

(6) Nutrient agar medium

It grows vigorously. Colonies are of a pale orange color, smooth and glossy. Some colonies are smooth while others are rough.

(7) Yeast-malt agar medium

It grows vigorously. Colonies are of an orange color, wrinkly and glossy. Some colonies are slimy.

(8) Oatmeal agar medium

It grows moderately well. Colonies are of a pale orange color and dimly glossy.

(C) Physiological properties

(i) Growth range

| | |
|---|---|
| Temperature: | 15 to 37 C, preferably 25 to 35 C |
| Medium pH value: | 5 to 9.5 preferably 6 tc 8 |

(2) Liquefaction of gelatin (glucose-peptone-gelatin medium)

Negative.

(3) hydrolysis of starch (starch agar medium)

Negative.

(4) Coagulation and peptonization of skim-milk

Both negative.

(5) Melanogenesis (tyrosine medium and pepton-yeast-iron medium)

Negative.

(D) Utilization of carbon sourse (Pridham-Gotreeve agar medium)

(1) L-Arabinose: +.
(2) D-Xylose: +.
(3) D-Glucose: +.
(4) D-Fructose: +.
(5) Sucrose: +.
(6) Inositol: +.
(7) L-Rhamnose: +.
(8) D-Mannitol: +.

(E) Chemotaxonomic properties

(1) Glycolyl test

Glycolyl type.

(2) Menaquinone system

MK-8 ($H_2$).

Based on these mycological properties, it has been recognized that this strain belongs to the genus Rhodococcus according to Bergey's Manual of Systematic Bacteriology, vol. 2 (1986). However it is significantly different from other microorganisms belonging to the genus Rhodococcus in unsaturated fatty acid productivity. Thus it was named Rhodococcus sp. KSM-B-3M and was deposited with Fermentation Research Institute, Agency of Industrial Science and Technology, Ministry of International Trade and Industry as FERM BP-1531. on October 21, 1987.

The fatty acid or derivative thereof to be used as the starting material in the process of the present invention are those represented by the following general formula (I):

$R\text{-}COOR_1$ (I)

wherein R represents a straight-chain or branched and saturated or unsaturated hydrocarbon group carrying 1 to 22 carbon atoms: and
$R_1$ represents a hyrogen atom, a straight-chain or branched hydrocarbon group or an aromatic hydrocarbon group carrying 1 to 10 carbon atoms, an alkali metal atom or a group of the formula:

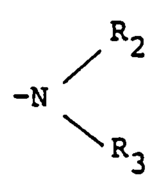

wherein $R_2$ and $R_3$ represent each a hydrogen atom or a straight-chain or branched hydrocarbon group with one to five carbon atoms.

In the present invention, it is preferable that R is a straight-chain hydrocarbon group with 14 to 20 carbon atoms and $R_1$ is a straight-chain or branched hydrocarbon group with two to four carbon atoms.

According to the present invention, the above fatty acid or a derivative thereof is converted into the corresponding unsaturated fatty acid or derivative thereof. More particularly, methyl, ethyl, n-propyl, isopropyl, n-butyl, isobutyl and tert-butyl esters of fatty acids such as n-hexadecanoic or n-tetradecanoic acid may be converted into methyl, ethyl, n-propyl, isopropyl, n-butyl, isobutyl and tert-butyl esters of hexadecenoic or tetradecenoic acid, respectively.

The hydrocarbon or derivative thereof to be used as the starting material in the process of the present invention are those represented by the following general formula (II):

R-A (II)

wherein R represents a straight-chain or branched and saturated or unsaturated hydrocarbon group with 6 to 22 carbon atoms; and

A represents a hydrogen atom, an aromatic hydrocarbon group, a halogen atom or a -OH, -CN, -CO-$R_1$ (wherein $R_1$ represents a straight-chain or branched hydrocarbon group or an aromatic hydrocarbon group with 1 to 10 carbon atoms), -O-$R_2$ (wherein $R_2$ represents a straight-chain or branched hydrocarbon group with one to ten carbon atoms or an aromatic hydrocarbon group), a metal atom or a group of the formula:

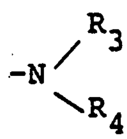

wherein $R_3$ and $R_4$ represent each a hydrogen atom or a straight-chain or branched hydrocarbon group with one to five carbon atoms.

In the present invention, it is preferable that R is a straight-chain hydrocarbon group carrying 14 to 20 carbon atoms.

According to the present invention, the above hydrocarbon or a derivative thereof is converted into the corresponding unsaturated hydrocarbon or derivative thereof. More particularly, hydrocarbons and derivatives thereof such as hexadecane, octadecane, tetradecane, pentadecane, eicosane, chlorohexadecane, chlorooctadecane, hexadecanol, hexadecylbenzene, heptadecanonitrile, hexadecene, eicosene and tetradecene are converted into hexadecene, octadecene, tetradecene, eicosene, chlorohexadecene, chlorooctadecene, hexadecenol, hexadecenylbenzene, heptadecenonitrile, hexadecadiene, eicosadiene and tetradedadiene, respectively.

In the present invention, cells of the abovementioned strain may be obtained by a conventional microbial culture method. The medium, pH, temperature and period for the culture may be arbitrarily selected so long as the strain can grow thereunder. It is preferred to culture this strain at 30°C for one to two days under aerobic conditions.

The culture medium thus obtained may be subjected to the following reaction either as such or after collecting cells by, for example, centrifugation or filtration.

0.1 to 90%, preferably 0.2 to 0.5%,in terms of dry cells, of the culture medium or cells obtained above are suspended in water or a buffer solution (pH 6 to 8). 1 to 70%, preferably 10 to 30%, of a fatty acid or a hydrocarbon to react and 0.05 to 10%, preferably 0.5 to 2%, of an amino acid such as L-glutamic acid or L-aspartic acid or a carbohydrate such as glucose are added to the above suspension to thereby give a basic reaction mixture. Further 0.001 to 2%, preferably 0.01 to 0.3% in terms of thiamine, of a thiamine compound such as thiamine hydrochloride or 0.001 to 2% of a magnesium or manganese salt such as magnesium sulfate or manganese sulfate, preferably 0.01 to 0.3% of magnesium sulfate, are added thereto to thereby give the reaction mixture of the present invention. This reaction mixture is allowed to react at 20 to 37°C, preferably 25 to 30°C, under aerobic conditions.

The addition of the thiamine or inorganic metal salt such as magnesium sulfate to the reaction mixture aims at significantly enhancing the productivity. Although it is preferable to combine these additives with an amino acid such as L-glutamic or L-aspartic acid, they may be combined with a carbohydrate. Alternately, each of these additives may be combined with an amino acid or a carbohydrate. These addtives may be added during the collection of the cells.

The thiamine may be employed either in the salt-free form or as hydrochloride or sulfate. Although magnesium sulfate is preferable as the inorganic metal salt, those including sulfuric acid, magnesium or

manganese such as manganese sulfate may be employed therefor.

Further an extract originating from a natural material or an amino acid mixture containing amino acids, carbohydrates, thiamine and inorganic metal salt(s), for example, meat extract, yeast extract and casamino acid may be employed.

When the reaction is to be conducted batchwise, the reaction period may range from one to three days. However the reaction period may be prolonged while supplying, for example, the starting fatty acid or its derivative, amino acid, vitamins and metal salt to the system.

It is also possible to recover the employed cells by, for example, centrifugation or filtration and then reuse the same.

This way the unsaturated hydrocarbon or fatty acid corresponding to the starting hydrocarbon or fatty acid is formed to achieve the purpose of the invention.

The product of the reaction can be identified and determined in a conventional manner employed for the identification and determination of organic compounds contained in natural materials. For example, a given amount of the reaction mixture is extracted with an organic solvent and analyzed by, for example, gas chromatography (GC) or gas chromatography/mass spectrometry (GC/MS) to thereby identify and determine the products. The position of a double bond in an unsaturated product may be determined by, for example, treating the mixture with dimethyl disulfide and then subjected to GC/MS, i.e., methylthioetherification (cf. Shibahara et al., Yukagaku, 34, 619 (1985)).

Alternatively, the reaction mixture may be centrifuged to thereby divide it into the cells, an oily phase and an aqueous phase and then the oily phase is collected. Alternatively, the reaction mixture is extracted with an organic solvent, either as such or after removing the cells by, for example, filtration. Thus the aimed compounds may be readily collected. The compounds thus collected may be further purified or isolated by, for example, conventional column chromatography or partition extraction, if required.

Brief Description of the Drawings:

Fig. 1 shows the GC/FT-IR spectrum of propyl hexadecenoate produced according to the present invention.

Fig. 2 shows the mass spectrum of the bismethylthioether derivative of propyl hexadecenoate produced according to the present invention.

Fig. 3 shows the mass spectrum of propyl hexadecenoate produced according to the present invention.

Fig. 4 shows the GC/FT-IR spectrum of cis-hexadecene produced according to the present invention.

Fig. 5 shows the mass spectrum of the bismethylthioether derivative of cis-hexadecene produced according to the present invention.

Fig. 6 shows the mass spectrum of cis-hexadecene produced according to the present invention.

Referential Example 1

Approximately 0.5 g of a soil sample collected in Okinawa pref. was suspended in 10 ml of sterilized water. After thoroughly stirring, 0.2 ml of the suspension was inoculated into 10 ml of a liquid medium (I) of the following composition placed in a test tube (25 mm in diameter x 200 mm in height) and cultured therein at 30 ° C for four days under shaking.

| Liquid medium (I): | |
|---|---|
| n-hexadecane | 100 g |
| $(NH_4)_2SO_4$ | 20 g |
| $KH_2PO_4$ | 20 g |
| yeast extract | 2 g |
| $MgSO_4 \cdot 7H_2O$ | 0.5 g |
| $FeSO_4 \cdot 7H_2O$ | 0.01 g |
| $MnSO_4 \cdot 4{\sim}6H_2O$ | 0.008 g |
| deionized water | 1 ℓ |
| pH | 7 |

The culture medium thus obtained was appropriately diluted with sterilized water, transplanted into a common agar medium (mfd. by Eiken Kagaku K.K.) and cultured therein at 30 ° C for two days. The transplantation to the nutrient agar medium was repeated until formed colonies were the same with each

other when observed with the naked eye and under a microscope.

The strain thus isolated was suspended in a 0.1 M phosphate buffer solution (pH 7.0) and irradiated with UV light for 90 sec. Then the colonies thus obtained were transplanted into 50 ml of a medium comprising 2.5 g of glucose, 17 g of Polypeptone, 3 g of Polypeptone S, 2.5 g of monopotassium dihydrogenphosphate, 5 g of sodium chloride and 1 ℓ of deionized water contained in a 500 ml Sakaguchi flask, tradename, and subsequently cultured therein at 30°c for one day under shaking. This culture medium was centrifuged to thereby collect cells, which were then suspended in 19 ml of a 0.25 M phosphate buffer solution (pH 7.0) containing 0.5% of glucose. After adding 1 ml of hexadecane, these cells were reacted in a 500 ml Sakaguchi flask at 30°C for three days under shaking. After the completion of the reaction, the major product contained in the reaction mixture was extracted and analyzed by, for example, gas chromatography. Thus a strain producing hexadecene was obtained.

This hexadecene-producing strain was appropriately diluted with sterilized water, transplanted into a nutrient agar medium and cultured therein at 30°c for two days. Then it was repeatedly transplanted into the nutrient agar medium until the colonies thus formed were the same with each other when observed with the naked eye and under a microscope.

Ten of the colonies grown in the nutrient agar medium were inoculated into each a slant agar medium (II) and cultured therein at 30°C for three days. Thus it was confirmed that the strains on these ten media were the same with each other by observing them with the naked eye and under a microscope. It was further confirmed that these ten strains were the same with each other in morphological and physiological properties.

| Slant agar medium (II): | |
|---|---|
| n-hexadecane | 20 g |
| $(NH_4)_2SO_4$ | 20 g |
| $KH_2PO_4$ | 2 g |
| yeast extract | 2 g |
| $MgSO_4 \cdot 7H_2O$ | 0.5 g |
| $FeSO_4 \cdot 7H_2O$ | 0.01 g |
| $MnSO_4 \cdot 4\sim6H_2O$ | 0.008 g |
| polyoxyethylene sorbitan monolaurate (average EO number: 20 mol) | 0.05 g |
| agar | 20 g |
| deionized water | 1 ℓ |
| pH | 7 |

Then a loopful of each strain above obtained was suspended in 2 ml of a sterilized 10% aqueous solution of glycerol contained in a cryopreservation vial and cryopreserved at -80°C.

Three months thereafter, it was rapidly thawed and a loopful of the reserved suspension was regenerated in an agar medium. Then it was confirmed that the morphological and physiological properties thereof on various media showed no change under the same conditions as those employed above.

The cryopreservation and thawing were repeated five times at intervals of one month. Then the strains showed no change in morphological and physiological properties on various media.

The morphological and physiological properties of this strain on various media are as described above.

(Examples)

To further illustrate the present invention, the following Examples will be given.

Example 1

50 ml of a medium comprising 2.5 g of glucose, 17 g of Polypeptone (mfd. by Daigo Eiyo K.K.), 3 g of Polypeptone S (mfd. by Daigo Eiyo K.K.), 2.5 g of monopotassium dihydrogenphosphate, 5 g of sodium chloride and 1 ℓ of deionized water was introduced into a 500-ml Sakaguchi flask and inoculated with Rhodococcus sp. KSM-B-3M strain. This strain was cultured in the medium at 30°C for one day under shaking. Then 1 ml of the culture medium was transplanted into 100 ml of a medium of the same composition as the one described above in a 500-ml Sakaguchi flask and cultured therein at 30°C for one day under shaking. The culture medium was centrifuged at 5,000 x g to thereby give 2 g of cells. 1 g of these cells were suspended in 20 ml of a 0.25 M phosphate buffer solution (pH 7.0) containing 1% of

monosodium glutamate, 0.1% of thiamine hydrochloride and 0.1% of magnesium sulfate. Then 4 ml of n-propyl palmitate (n-propyl hexadecanoate) was added thereto and the resulting mixture was allowed to react in a 500-ml Sakaguchi flask at 26°C for two days under shaking.

After the completion of the reaction, the major product contained in the reaction mixture was extracted with ethyl acetate and analyzed by GC, GC/MS or gas chromatography/infrared spectrocopy (GC/FT-IR) (cf. Fig. 1). Thus it was confirmed that the major product was propyl cis-6-hexadecenoate. The position of the double bond was determined by converting the product into a dimethyl disulfide derivative and analyzing the same with mass spectroscopy (cf. Fig. 2). Fig. 3 shows the mass spectrum of the major product.

1 ml of the reaction mixture was extracted with 3 ml of ethyl acetate and the content of the propyl cis-6-hexadecenoate therein was determined by gas chromatography. As a result, the productivity of this strain of the above compound was found to be 15 g/l•2 days.

Example 2

The procedure of Example 1 was repeated except that the propyl hexadecanoate was replaced with methyl hexadecanoate, ethyl hexadecanoate, isopropyl hexadecanoate, isobutyl hexadecanoate and n-butyl hexadecanoate.

The unsaturated fatty acid ester in each reaction mixture was identified and determined in the same manner as the one described in Example 1. Table 1 shows the results.

Table 1

| Substrate | Major product | Production g/l•2 days |
|---|---|---|
| methyl hexadecanoate | methyl cis-9-hexadecenoate | 0.5 |
| ethyl hexadecanoate | ethyl cis-6-hexadecenoate | 8.5 |
| iso-propyl hexadecanoate | iso-propyl cis-6-hexadecenoate | 28.0 |
| iso-butyl hexadecanoate | iso-butyl cis-6-hexadecenoate | 10.4 |
| n-butyl hexadecanoate | n-butyl cis-4-hexadecenoate | 5.4 |

Example 3

The procedure of Example 1 was repeated except that the propyl hexadecanoate was replaced with sodium hexadecanoate, isopropyl tetradecanoate and ethyl tetradecanoate.

The unsaturated fatty acid or its ester in each reaction mixture was identified and determined in the same manner as the one described in Example 1. Table 2 shows the results.

Table 2

| Substrate | Major product | Production g/l•2 days |
|---|---|---|
| sodium hexadecanoate | hexadecenoic acid | 0.2 |
| iso-propyl tetradecanoate | iso-propyl tetradecenoate | 10.5 |
| ethyl tetradecanoate | ethyl tetradecenoate | 2.1 |

Comparative Example 1

50 ml of a medium comprising 2.5 g of glucose, 17 g of Polypeptone, 3 g of Polypeptone S, 2.5 g of monopotassium dihydrogenphosphate, 5 g of sodium chloride and 1 ℓ of deionized water was introduced into a 500-ml Sakaguchi flask and inoculated with Rhodococcus sp. KSM-B-3M strain. This strain was cultured in the medium at 30°C for one day under shaking. Then 1 ml of the culture medium was transplanted into 100 ml of a medium of the same composition as the one described above in a 500-ml Sakaguchi flask and cultured therein at 30°C for one day under shaking. The culture medium was centrifuged at 5,000 x g to thereby give 2 g of cells. 1 g of these cells were suspended in 20 ml of a 0.25 M phosphate buffer solution (pH 7.0) containing 1.0% of glucose. Then 4 ml of n-propyl hexadecanoate was

added thereto and the resulting mixture was allowed to react in a 500-ml Sakaguchi flask at 26 °C for two days under shaking.

1 ml of the reaction mixture was extracted vith 3 ml of ethyl acetate and the propyl hexadecenoate thus formed was identified and determined in the same manner as the one described in Example 1. As a result, the productivity of this strain of the above compound was found to be 4.0 g/l•2 days.

Comparative Example 2

The procedure of Comparative Example 1 was repeated except that the 0.25 M phosphate buffer solution containing 1.0% of glucose was replaced with a 0.25 M phosphate buffer solution containing 1.0% of monosodium L-glutamate.

The isopropyl hexadecenoate thus formed in the reaction mixture was identified and determined in the same manner as the one described in Example 1. As a result, the productivity of this strain of the above compound was found to be 5.2 g/l•2 days.

Example 4

The procedure of Comparative Example 1 was repeated except that the 0.25 M phosphate buffer solution containing 1.0% of glucose was replaced with:

(1) a 0.25 M phosphate buffer solution containing 1.0% of glucose and 0.1% of thiamine hydrochloride:

(2) a 0.25 M phosphate buffer solution containing 1.0% of monosodium L-glutamate and 0.1% of magnesium sulfate:

(3) a 0.25 M phosphate buffer solution containing 1.0% of monosodium L-glutamate and 0.1% of thiamine hydrochloride:

(4) a 0.25 M phosphate buffer solution containing 1.0% of monosodium L-glutamate, 0.1% of thiamine hydrochloride and 0.1% of magnesium sulfate:

(5) a 0.25 M phosphate buffer solution containing 1.0% of monosodium L-aspartate, 0.1% of thiamine hydrochloride and 0.1% of magnesium sulfate:

(6) a 0.25 M phosphate buffer solution containing 1.0% of glycine and 0.1% of thiamine hydrochloride: 0.1% of magnesium sulfate:

(7) a 0.25 M phosphate buffer solution containing 1.0% of L-tyrosine, 0.1% of thiamine hydrochloride and 0.1% of magnesium sulfate:

(8) a 0.25 M phosphate buffer solution containing 1.0% of L-threonine, 0.1% of thiamine hydrochloride and 0.1% of magnesium sulfate:

(9) a 0.25 M phosphate buffer solution containing 1.0% of L-proline, 0.1% of thiamine hydrochloride and 0.1% of magnesium sulfate: and

(10) a 0.25 M phosphate buffer solution containing 1.0% of monosodium L-glutamate and 0.1% of manganese sulfate.

The unsaturated fatty acid ester in each reaction mixture was identified and determined in the same manner as the one described in Example 1. Table 3 shows the results.

Example 5

50 ml of a medium comprising 2.5 g of glucose, 17 g of Polypeptone (mfd. by Daigo Eiyo K.K.), 3 g of Polypeptone S (mfd. by Daigo Eiyo K.K.), 2.5 g of monopotassium dihydrogenphosphate, 5 g of sodium chloride and 1 ℓ of deionized water was introduced into a 500-ml Sakaguchi flask and inoculated with Rhodococcus sp. KSM-B-3M strain. This strain was cultured in the medium at 30 °C for one day under shaking. Then 1 ml of the culture medium was transplanted into 100 ml of a medium of the same composition as the one described above in a 500-ml Sakaguchi flask and cultured therein at 30 °C for one day under shaking. The culture medium was centrifuged at 5,000 x g to thereby give 2 g of cells. 1 g of these cells were suspended in 20 ml of a 0.25 M phosphate buffer solution (pH 7.0) containing 1% of monosodium glutamate, 0.1% of thiamine hydrochloride and 0.1%

Table 3

| | No. | Reaction condition | Production of isopropyl hexadecenoate (g/1·2 days) | Productivity |
|---|---|---|---|---|
| Comparison | | 1.0% glucose | 4.0 | x |
| | | 1.0% monosodium L-glutamate | 5.2 | x |
| Invention | (1) | 1.0% glucose, 0.1% thiamine hydrochloride | 10.3 | ○ |
| | (2) | 1.0% monosodium L-glutamate, 0.1% magnesium sulfate | 10.1 | ○ |
| | (3) | 1.0% monosodium L-glutamate, 0.1% thiamine hydrochloride | 15.5 | ○ |
| | (4) | 1.0% monosodium L-glutamate, 0.1% thiamine hydrochloride, 0.1% magnesium sulfate | 28.2 | ◎ |
| | (5) | 1.0% monosodium L-aspartate, 0.1% thiamine hydrochloride, 0.1% magnesium sulfate | 28.4 | ◎ |
| | (6) | 1.0% glycine, 0.1% thiamine hydrochloride, 0.1% magnesium sulfate | 22.7 | ◎ |
| | (7) | 1.0% L-tyrosine, 0.1% thiamine hydrochloride, 0.1% magnesium sulfate | 27.7 | ◎ |
| | (8) | 1.0% L-threonine, 0.1% thiamine hydrochloride, 0.1% magnesium sulfate | 25.3 | ◎ |
| | (9) | 1.0% L-proline, 0.1% thiamine hydrochloride, 0.1% magnesium sulfate | 26.9 | ◎ |
| | (10) | 1.0% monosodium L-glutamate, 0.1% manganese sulfate | 8.5 | ○ |

of magnesium sulfate. Then 5 ml of n-hexadecane was added thereto and the resulting mixture was allowed to react in a 500-ml Sakaguchi flask at 30 ° C for two days under shaking.

After the completion of the reaction, the major product contained in the reaction mixture was extracted with ethyl acetate and analyzed by GC, GC/MS or gas chromatography/infrared spectrocopy (CG/FT-IR) (cf. Fig. 4). Thus it was confirmed that the major product was cis-7-hexadecene. The position of the double

bond was determined by converting the product into a dimethyl disulfide derivative and analyzing th same with mass spectroscopy (cf. Fig. 5). Fig. 6 shows the mass spectrum of the major product.

1 ml of the reaction mixture was extracted vith 3 ml of ethyl acetate and the content of the cis-7-hexadecene therein was determined by gas chromatography. As a result, the productivity of this strain of the above compound was found to be 18 g/l•2 days.

Example 6

The procedure of Example 5 was repeated except that the n-hexadecane was replaced with n-dodecane, n-tetradecane, n-octadecane, n-eicosane, 1-chlorotetradecane, 1-chlorohexadecane, 1-chlorooctadecane and 1-chloroeicosane. The unsaturated hydrocarbon or its derivative in each reaction mixture was identified and determined in the same manner as the one described in Example 5. Table 4 shows the results.

Table 4

| Substrate | Major product | Production g/l•2 days |
|---|---|---|
| n-dodecane | cis-3-dodecene | 2.1 |
| n-tetradecane | cis-5-tetradecene | 8.3 |
| n-octadecane | cis-9-octadecene | 16.2 |
| n-eicosane | cis-11-eicosene | 10.6 |
| 1-chloro-tetradecane | 1-chloro-cis-5-tetradecene | 7.2 |
| 1-chloro-hexadecane | 1-chloro-cis-7-hexadecene | 25.6 |
| 1-chloro-octadecane | 1-chloro-cis-9-octadecene | 22.5 |
| 1-chloro-eicosane | 1-chloro-cis-11-eicosene | 15.2 |

Example 7

The procedure of Example 5 was repeated except that the n-hexadecane was replaced with n-hexadecanol, n-hexadecylbenzene and n-heptadecanonitrile.

The unsaturated hydrocarbon in each reaction mixture was identified and determined in the same manner as the one described in Example 5. Table 5 shows the results.

Table 5

| Substrate | Major product | Production g/l•2 days |
|---|---|---|
| 1-hexadecanol | hexadecenol | 0.4 |
| n-hexadecylbenzene | hexadecenyl benzene | 0.4 |
| n-heptadecanonitorile | heptadecenonitorile | 6.0 |

Example 8

The procedure of Example 5 was repeated except that the n-hexadecane was replaced with 1-hexadecene, 1-octadecene, 1-eicosene and 1-tetradecene.

The unsaturated hydrocarbon in each reaction mixture was identified and determined in the same manner as the one described in Example 5. Table 6 shows results.

Table 6

| Substrate | Major product | Production g/l•2 days |
|---|---|---|
| 1-hexadecene | hexadeca-di-ene | 22.7 |
| 1-octadecene | octadeca-di-ene | 20.1 |
| 1-eicosene | eicosa-di-ene | 19.3 |
| 1-tetradecene | tetradeca-di-ene | 7.2 |

Comparative Example 3

50 ml of a medium comprising 2.5 g of glucose, 17 g of Polypeptone, 3 g of Polypeptone S, 2.5 g of monopotassium dihydrogenphosphate, 5 g of sodium chloride and 1 ℓ of deionized water was introduced into a 500-ml Sakaguchi flask and inoculated with Rhodococcus sp. KSM-B-3M strain. This strain was cultured in the medium at 30°C for one day under shaking. Then 1 ml of the culture medium was transplanted into 100 ml of a medium of the same composition as the one described above in a 500-ml Sakaguchi flask and cultured therein at 30°C for one day under shaking. The culture medium was centrifuged at 5,000 x g to thereby give 2 g of cells. 1 g of these cells were suspended in 20 ml of a 0.25 M phosphate buffer solution (pH 7.0) containing 1.0% of glucose. Then 4 ml of 1-chlorohexadecane was added thereto and the resulting mixture was allowed to react in a 500-ml Sakaguchi flask at 30°C for two days under shaking.

1 ml of the reaction mixture was extracted with 3 ml of ethyl acetate and the 1-chlorohexadecene thus formed was identified and determined in the same manner as the one described in Example 1. As a result, the productivity of this strain of the above compound was found to be 5.5 g/l•2 days.

Comparative Example 4

The procedure of Comparative Example 3 was repeated except that the 0.25 M phosphate buffer solution containing 1.0% of glucose was replaced with a 0.25 M phosphate buffer solution containing 1.0% of monosodium L-glutamate.

The 1-chlorohexadecene thus formed in the reaction mixture was identified and determined in the same manner as the one described in Example 3. As a result, the productivity of this strain of the above compound was found to be 6.2 g/l•2 days.

Example 9

The procedure of Comparative Example 3 was repeated except that the 0.25 M phosphate buffer solution containing 1.0% of glucose was replaced with:

(1) a 0.25 M phosphate buffer solution containing 1.0% of monosodium L-glutamate and 0.1% of magnesium sulfate;

(2) a 0.25 M phosphate buffer solution containing 1.0% of glucose and 0.1% of thiamine hydrochloride;

(3) a 0.25 M phosphate buffer solution containing 1.0% of monosodium L-glutamate and 0.1% of thiamine hydrochloride:

(4) a 0.25 M phosphate buffer solution containing 1.0% of monosodium L-glutamate, 0.1% of thiamine hydrochloride and 0.1% of magnesium sulfate:

(5) a 0.25 M phosphate buffer solution containing 1.0% of monosodium L-aspartate, 0.1% of thiamine hydrochloride and 0.1% of magnesium sulfate:

(6) a 0.25 M phosphate buffer solution containing 1.0% of glycine, 0.1% thiamine hydrochloride and 0.1% of magnesium sulfate:

(7) a 0.25 M phosphate buffer solution containing 1.0% of L-tyrosine, 0.1% of thiamine hydrochloride and 0.1% of magnesium sulfate:

(8) a 0.25 M phosphate buffer solution containing 1.0% of L-threonine, 0.1% of thiamine hydrochloride and 0.1% of magnesium sulfate:

(9) a 0.25 M phosphate buffer solution containing 1.0% of L-proline, 0.1% of thiamine hydrochloride and 0.1% of magnesium sulfate: and

(10) a 0.25 M phosphate buffer solution containing 1.0% of monosodium L-glutamate and 0.1% of manganese sulfate.

The 1-chlorohexadecene in each reaction mixture was identified and determined in the same manner as the one described in Example 5. Table 7 shows results.

Table 7

| | No. | Reaction condition | Production of 1-chloro-hexadecene (g/l·2 days) | Productivity |
|---|---|---|---|---|
| Comparison | | 1.0% glucose | 5.5 | x |
| | | 1.0% monosodium L-glutamate | 6.2 | x |
| Invention | (1) | 1.0% monosodium L-glutamate, 0.1% magnesium sulfate | 10.8 | ○ |
| | (2) | 1.0% glucose, 0.1% thiamine hydrochloride | 10.4 | ○ |
| | (3) | 1.0% monosodium L-glutamate, 0.1% thiamine hydrochloride | 11.5 | ○ |
| | (4) | 1.0% monosodium L-glutamate, 0.1% thiamine hydrochloride, 0.1% magnesium sulfate | 25.6 | ◎ |
| | (5) | 1.0% monosodium L-aspartate, 0.1% thiamine hydrochloride, 0.1% magnesium sulfate | 25.5 | ◎ |
| | (6) | 1.0% glycine, 0.1% thiamine hydrochloride, 0.1% magnesium sulfate | 19.4 | ◎ |
| | (7) | 1.0% L-tyrosine, 0.1% thiamine hydrochloride, 0.1% magnesium sulfate | 20.1 | ◎ |
| | (8) | 1.0% L-threonine, 0.1% thiamine hydrochloride, 0.1% magnesium sulfate | 21.1 | ◎ |
| | (9) | 1.0% L-proline, 0.1% thiamine hydrochloride, 0.1% magnesium sulfate | 22.8 | ◎ |
| | (10) | 1.0% monosodium L-glutamate, 0.1% manganese sulfate | 0.0 | ○ |

## Claims

**1.** A process for preparing:

(i) an unsaturated fatty acid or a derivative thereof from a fatty acid or derivative thereof represented by the general formula (I):

$R\text{-}COOR_1$ (I)

wherein R represents a straight-chain or branched and saturated or unsaturated hydrocarbon group with 1 to 22 carbon atoms: and

$R_1$ represents a hydrogen atom, a straight-chain or branched hydrocarbon group or an aromatic hydrocarbon group with 1 to 10 carbon atoms, an alkali metal atom or a group of the formula:

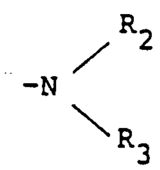

wherein each of $R_2$ and $R_3$ represents a hydrogen atom or a straight-chain or branched hydrocarbon group with one to five carbon atoms,

or (ii) an unsaturated hydrocarbon or a derivative thereof from a hydrocarbon or derivative thereof represented by the general formula (II):

R-A (II)

wherein R represents a straight-chain or branched saturated or unsaturated hydrocarbon group with 6 to 22 carbon atoms; and

A represents a hydrogen atom, an aromatic hydrocarbon group, a halogen atom or a -OH, -CN, $-CO\text{-}R_1$ (wherein $R_1$ represents a straight-chain or branched hydrocarbon group or an aromatic hydrocarbon group with 1 to 10 carbon atoms), or

$-O\text{-}R_2$ (wherein $R_2$ represents a straight-chain or branched hydrocarbon group with one to ten carbon atoms or an aromatic hydrocarbon group), or a metal atom or a group of the formula:

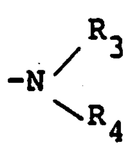

wherein each of $R_3$ and $R_4$ represents a hydrogen atom or a straight-chain or branched hydrocarbon group with one to five carbon atoms, wherein the process is carried out in the presence of Rhodococcus sp. KSM-B-3M strain (FERM-BP-1531),

characterised by conducting the reaction by suspending 0.1 to 90% of dry cells in water or a buffer solution at a pH of 6 to 8, adding 1 to 70% of the fatty acid or derivative thereof or the hydrocarbon or derivative thereof and 0.05 to 10% of an amino acid or carbohydrate to give a basic reaction mixture, adding 0.001 to 2% or thiamine or a thiamine compound or a magnesium or manganese salt, and allowing the resulting mixture to react at 20 to 37 °C under aerobic conditions.

**2.** A process as claimed in claim 1, in which said carbohydrate is selected from glucose, sorbitol, arabinose, xylose, fructose, inositol, rhamnose and mannitol.

**3.** A process as claimed in claim 1, in which said amino acid is selected from L-glutamic acid, L-aspartic acid, glycine, L-threonine, L-tyrosine, L-isoleucine and L-proline.

**4.** A process as claimed in claim 1, in which said magnesium or manganese salt is a sulphate.

5. A process as claimed in claim 1, in which said hydrocarbon or derivative thereof is selected from n-dodecane, n-heptadecane, n-hexadecane, n-octadecane, n-tetradecane, n-pentadecane, n-eicosane, chloroheptadecane, chlorohexadecane, chlorooctadecane, hexadecylalcohol, hexadecylbenzene, heptadecanonitrile, hexadecene, eiscosene, octadecene and tetradecene.

6. A process as claimed in claim 1, in which said fatty acid or derivative thereof is selected from n-heptadecanoic acid, n-hexadecanoic acid, n-pentadecanoic acid and n-tetradecanoic acid as well as methyl, ethyl, n-propyl, isopropyl, n-butyl, isobutyl, tertbutyl, n-heptyl and n-hexyl esters thereof.

7. A process according to claim 1, wherein the Rhodococcus sp.KSM-B-3M strain is that deposited at the Fermentation Research Institute, Japan, under the accession number FERM-BP-1531.

**Patentansprüche**

1. Verfahren zur Herstellung von:
(i) einer ungesättigten Fettsäure oder deren Derivat aus einer Fettsäure oder deren Derivat, dargestellt durch die allgemeine Formel (I):

$R\text{-}COOR_1$ (I)

worin R eine geradkettige oder verzweigte und gesättigte oder ungesättigte Kohlenwasserstoffgruppe mit 1 bis 22 Kohlenstoffatomen bedeutet, und
$R_1$ ein Wasserstoffatom bedeutet, eine geradkettige oder verzweigte Kohlenwasserstoffgruppe oder eine aromatische Kohlenwasserstoffgruppe mit 1 bis 10 Kohlenstoffatomen, ein Alkalimetallatom oder eine Gruppe der Formel:

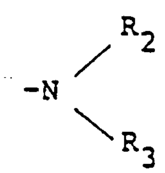

worin jedes $R_2$ und $R_3$ ein Wasserstoffatom oder eine geradkettige oder verzweigte Kohlenwasserstoffgruppe mit 1 bis 5 Kohlenstoffatomen bedeutet,
oder von
(ii) einem ungesättigten Kohlenwasserstoff oder dessen Derivat aus einem Kohlenwasserstoff oder dessen Derivat, dargestellt durch die allgemeine Formel (II):

R-A (II)

worin R eine geradkettige oder verzweigte gesättigte oder ungesättigte Kohlenwasserstoffgruppe mit 6 bis 22 Kohlenstoffatomen bedeutet; und
A ein Wasserstoffatom bedeutet, eine aromatische Kohlenwasserstoffgruppe, ein Halogenatom oder -OH, -CN, - $CO\text{-}R_1$ (worin $R_1$ eine geradkettige oder verzweigte Kohlenwasserstoffgruppe oder eine aromatische Kohlenwasserstoffgruppe mit 1 bis 10 Kohlenstoffatomen bedeutet), oder $-O\text{-}R_2$ (worin $R_2$ eine geradkettige oder verzweigte Kohlenwasserstoffgruppe mit 1 bis 10 Kohlenstoffatomen oder eine aromatische Kohlenwasserstoffgruppe bedeutet), oder ein Metallatom oder eine Gruppe der Formel:

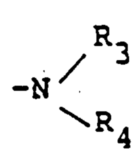

worin jedes $R_3$ und $R_4$ ein Wasserstoffatom oder eine geradkettige oder verzweigte Kohlenwasserstoffgruppe mit 1 bis 5 Kohlenstoffatomen bedeutet, wobei das Verfahren in Gegenwart von Rhodococcus sp. KSM-B-3M-Stamm (FERM-BP-1531) durchgeführt wird,

dadurch gekennzeichnet, daß die Reaktion durch Suspendieren von 0,1 bis 90% trockener Zellen in Wasser oder einer Pufferlösung bei einem pH von 6 bis 8, Zugeben von 1 bis 70% der Fettsäure oder deren Derivat oder des Kohlenwasserstoffs oder dessen Derivat und 0,05 bis 10% einer Aminosäure oder Carbohydrats zur Erzeugung einer basischen Reaktionsmischung, Zugeben von 0,001 bis 2% von Thiamin oder einer Thiaminverbindung oder einem Magnesium- oder Mangansalz und Umsetzen der resultierenden Mischung bei 20 bis 37 °C unter Aerobonbedingungen, durchgeführt wird.

**2.** Verfahren nach Anspruch 1, worin das Carbohydrat ausgewählt ist aus Glucose, Sorbit, Arabinose, Xylose, Fructose, Inosit, Rhamnose und Mannit.

**3.** Verfahren nach Anspruch 1, worin die Aminosäure ausgewählt ist aus L-Glutaminsäure, L-Aspartinsäure, Glycin, L-Threonin, L-Tyrosin, L-Isoleucin und L-Prolin.

**4.** Verfahren nach Anspruch 1, worin das Magnesium- oder Mangansalz ein Sulphat ist.

**5.** Verfahren nach Anspruch 1, worin der Kohlenwasserstoff oder dessen Derivat ausgewählt ist aus n-Dodecan, n-Heptadecan, n-Hexadecan, n-Octadecan, n-Tetradecan, n-Pentadecan, n-Eicosan, Chlorheptadecan, Chlorhexadecan, Chloroctadecan, Hexadecylalkohol, Hexadecylbenzol, Heptadecanonitril, Hexadecen, Eicosen, Octadecen und Tetradecen.

**6.** Verfahren nach Anspruch 1, worin die Fettsäure oder deren Derivat ausgewählt ist aus n-Heptadecansäure, n-Hexadecansäure, n-Pentadecansäure und n-Tetradecansäure ebenso wie deren Methyl-, Ethyl-, n-Propyl-, Isopropyl-, n-Butyl-, Isobutyl-, Tertbutyl-, n-Heptyl- und n-Hexylester davon.

**7.** Verfahren nach Anspruch 1, worin der Rhodococcus sp. KSM-B-3M-Stamm beim Fermentation Research Institute, Japan, unter der Hinterlegungsnr. FERM-BP-1531 hinterlegt ist.

## Revendications

**1.** Procédé pour la préparation de :

(i) un acide gras insaturé ou un dérivé de celui-ci à partir d'un acide gras ou d'un dérivé de celui-ci représenté par la formule générale (I) :

$R\text{-}COOR_1$ (I)

dans laquelle R représente un groupe hydrocarboné à chaîne linéaire ou ramifiée, saturé ou insaturé avec 1 à 22 atomes de carbone ; et

$R_1$ représente un atome d'hydrogène un groupe hydrocarboné à chaîne linéaire ou ramifiée ou un groupe hydrocarboné aromatique avec 1 à 10 atomes de carbone, un atome de métal alcalin ou un groupe de formule :

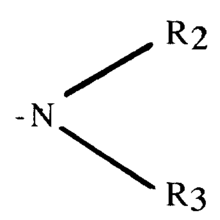

dans laquelle $R_2$ et $R_3$ représentent chacun un atome d'hydrogène ou un groupe hydrocarboné à chaîne linéaire ou ramifiée avec un à cinq atomes de carbone,

ou (ii) un hydrocarbure insaturé ou un dérivé de celui-ci à partir d'un hydrocarbure ou d'un dérivé de celui-ci représenté par la formule générale (II) :

R-A (II)

dans laquelle R représente un groupe hydrocarboné à chaîne linéaire ou ramifiée, saturé ou insaturé avec 6 à 22 atomes de carbone ; et

A représente un atome d'hydrogène, un groupe hydrocarboné aromatique, un atome d'halogène ou un -OH, -CN, -CO-$R_1$ (dans lequel $R_1$ représente un groupe hydrocarboné à chaîne linéaire ou ramifiée ou un groupe hydrocarboné aromatique avec 1 à 10 atomes de carbone), ou -O-$R_2$- (dans lequel $R_2$ représente un groupe hydrocarboné à chaîne linéaire ou ramifiée avec un à dix atomes de carbone ou un groupe hydrocarboné aromatique), ou un atome de métal ou un groupe de formule :

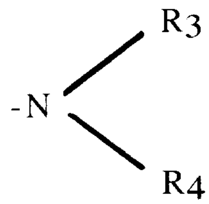

dans laquelle $R_3$ et $R_4$ représentent chacun un atome d'hydrogène ou un groupe hydrocarboné à chaîne linéaire ou ramifiée avec un à cinq atomes de carbone, lequel procédé est mis en oeuvre en présence de la souche Rhodococcus sp.KSM-B-3M (FERM-BP-1531),

caractérisé en ce que la réaction est mise en oeuvre en mettant en suspension 0,1 à 90 % de cellules sèches dans de l'eau ou dans une solution tampon à un pH compris entre 6 et 8, en ajoutant 1 à 70 % de l'acide gras ou du dérivé de celui-ci ou de l'hydrocarbure ou du dérivé de celui-ci et 0,05 à 10 % d'un acide aminé ou d'un hydrate de carbone pour obtenir un mélange réactionnel de base, en ajoutant 0,001 % à 2 % de thiamine ou d'un composé à base de thiamine ou d'un sel de magnésium ou de manganèse, et en laissant réagir le mélange résultant entre 20 et 37 ° C en conditions aérobies.

**2.** Procédé tel que revendiqué dans la revendication 1, dans lequel ledit hydrate de carbone est choisi parmi le glucose le sorbitol, l'arabinose, le xylose le fructose, l'inositol, le rhamnose et le mannitol.

**3.** Procédé tel que revendiqué dans la revendication 1, dans lequel ledit acide aminé est choisi parmi l'acide L-glutamique, l'acide L-aspartique, la glycine, la L-thréonine, la L-tyrosine, la L-isoleucine et la L-proline.

**4.** Procédé tel que revendiqué dans la revendication 1, dans lequel ledit sel de magnésium ou de manganèse est un sulfate.

**5.** Procédé tel que revendiqué dans la revendication 1, dans lequel ledit hydrocarbure ou dérivé de celui-ci est choisi parmi le n-dodécane, le n-heptadécane, le n-hexadécane, le n-octadécane, le n-tétradécane, le n-pentadécane, le n-eicosane, le chloroheptadécane, le chlorohexadécane, le chlorooctadécane, l'hexadécylalcool, l'hexadécylbenzène, l'heptadécanonitrile, l'hexadécène, l'eicosène, l'octadécène et le tétradécène.

**6.** Procédé tel que revendiqué dans la revendication 1, dans lequel ledit acide gras ou dérivé de celui-ci est choisi parmi l'acide n-heptadécanoïque l'acide n-hexadécanoïque l'acide n-pentadécanoïque et l'acide n-tétradécanoïque ainsi que des esters méthylique, éthylique, n-propylique, isopropylique, n-butylique, isobutylique, tert-butylique, n-heptylique et n-hexylique de ceux-ci.

**7.** Procédé tel que revendiqué dans la revendication 1, dans lequel la souche Rhodococcus sp. KSM-B-3M est celle déposée au Fermentation Research Institut, Japon, sous le numéro d'accès FERM-BP-1531.

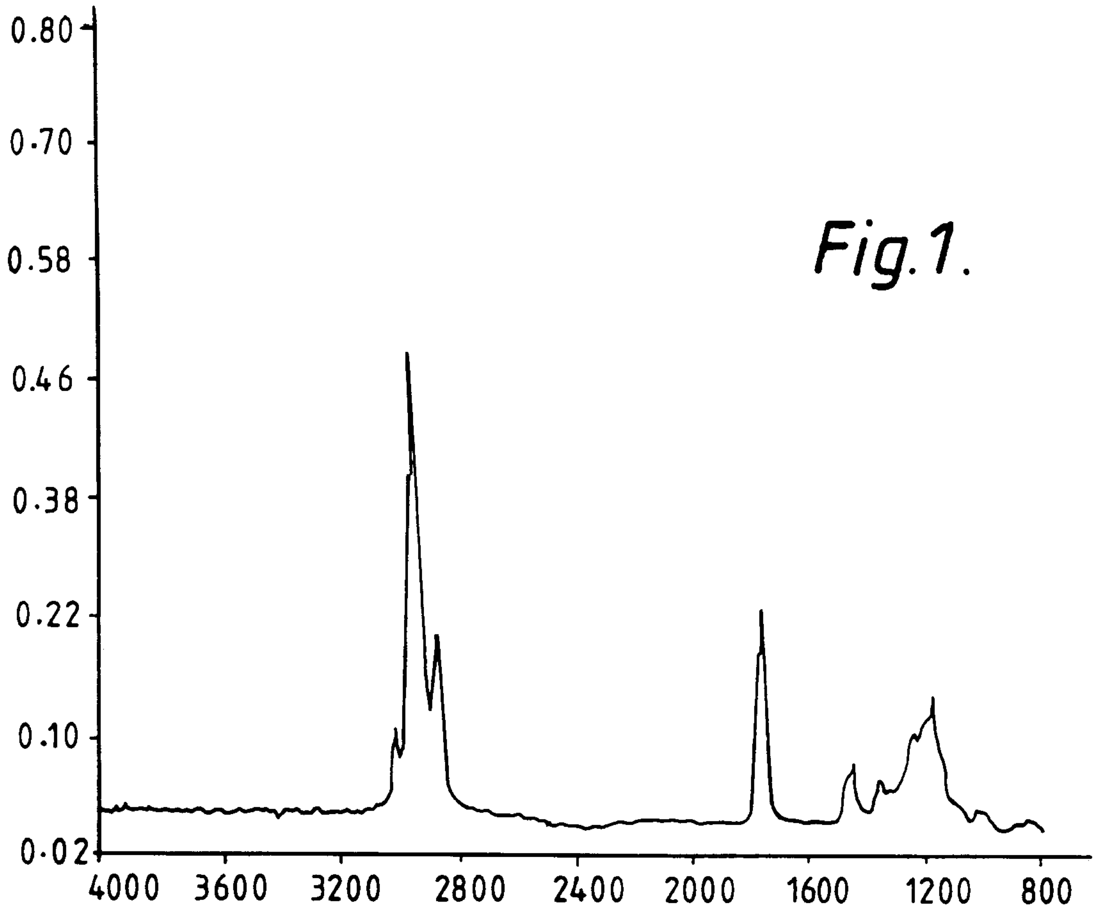
0.80
0·70
0.58
0.46
0.38
0.22
0.10
0·02
4000
3600
3200
2800
2400
2000
1600
1200
800

Fig.1.

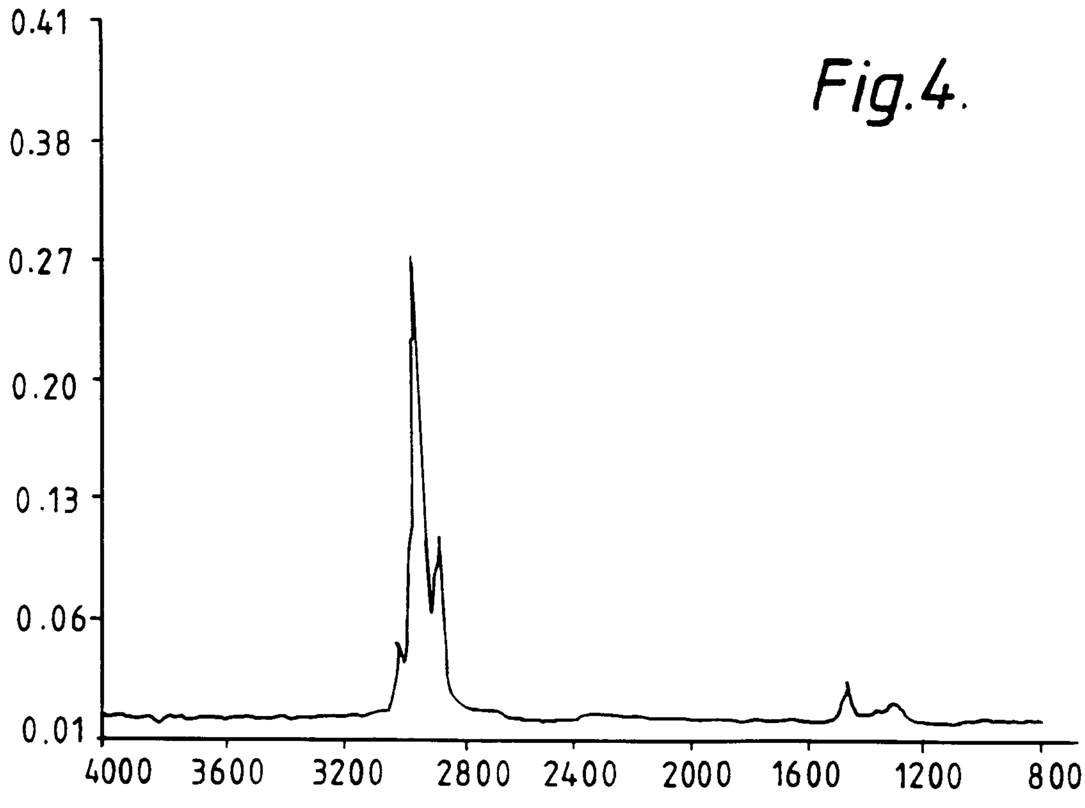
0.41
0.38
0.27
0.20
0.13
0.06
0.01
4000
3600
3200
2800
2400
2000
1600
1200
800

Fig.4.

Fig.2.

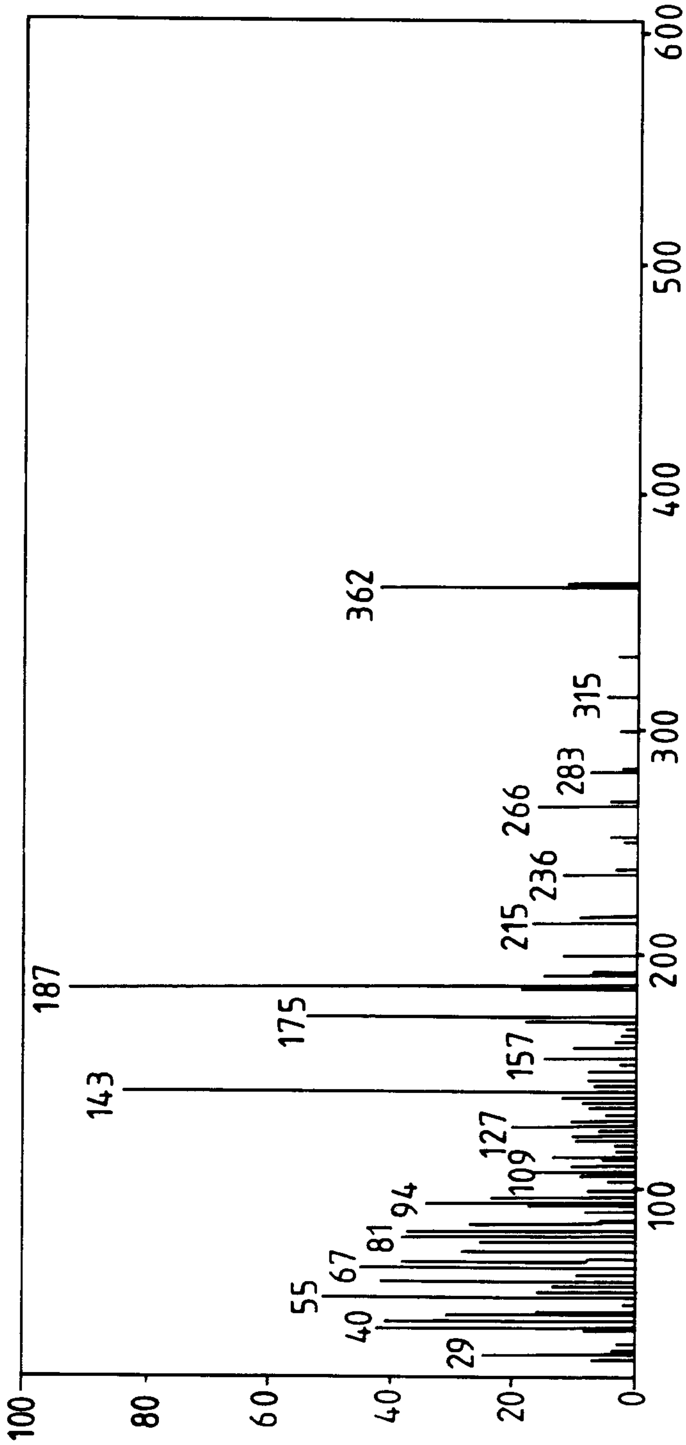
100
80
60
40
20
0
100
200
300
400
500
600
29
40
55
67
81
94
109
127
143
157
175
187
215
236
266
283
315
362

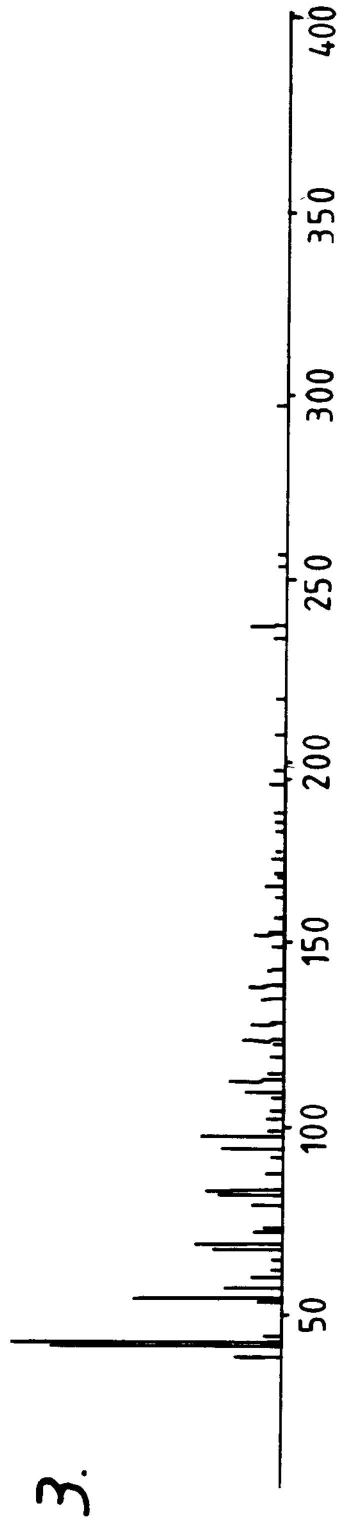
50
100
150
200
250
300
350
400

Fig.3.

Fig.5.

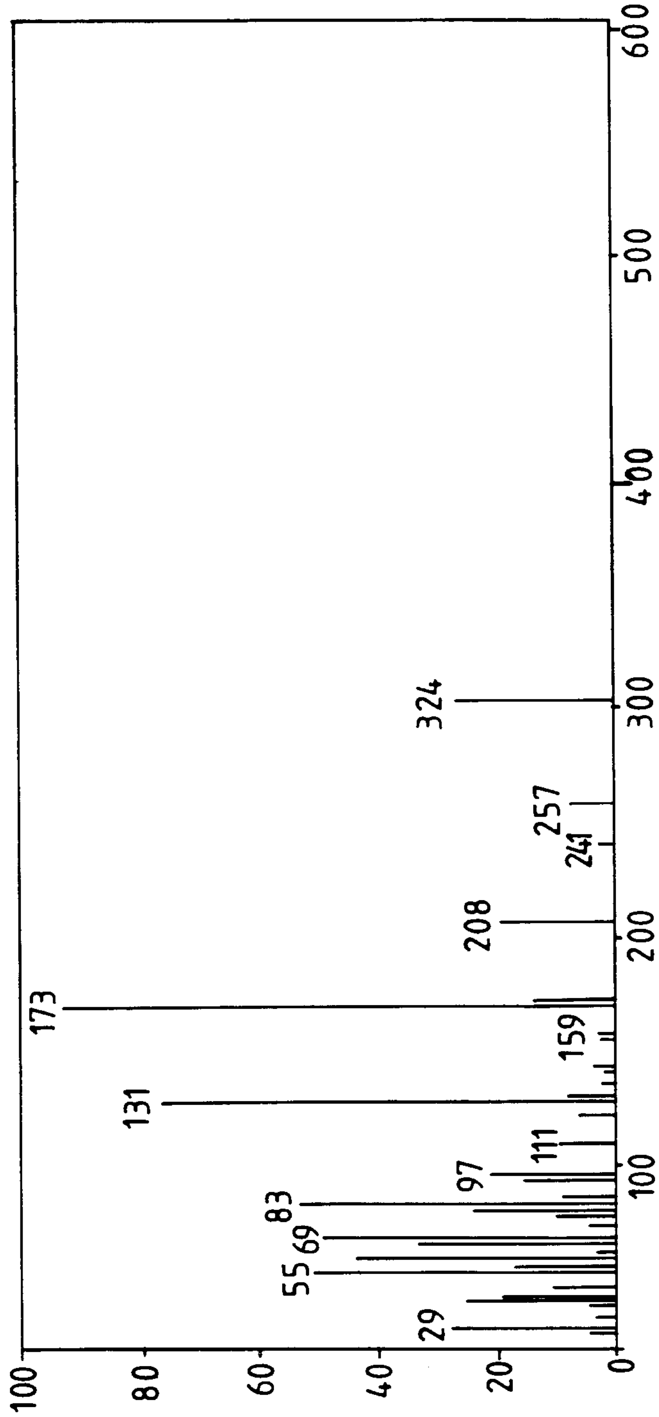
100
80
60
40
20
0
29
55
69
83
97
111
131
159
173
208
241
257
324
100
200
300
400
500
600

Fig.6.

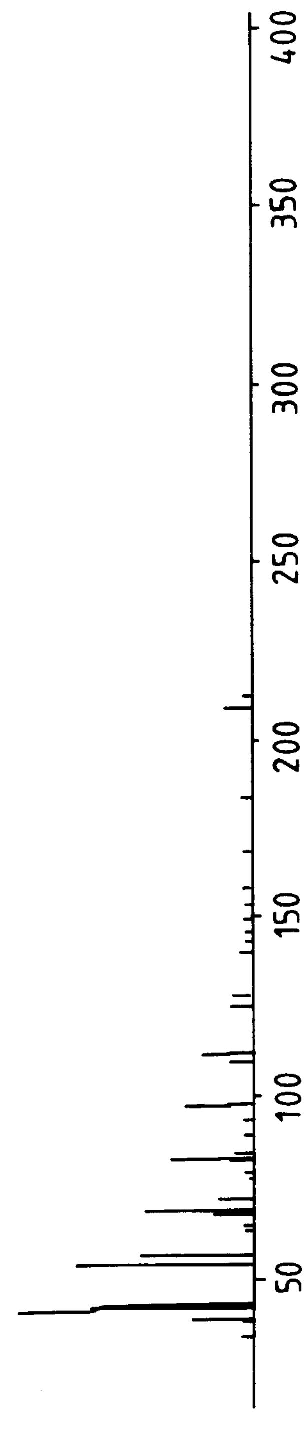
50
100
150
200
250
300
350
400